# EUROPEAN PATENT SPECIFICATION

(11) **EP 1 793 792 B1**
(45) Date of publication and mention of the grant of the patent: **29.02.2012**
(21) Application number: 04774293.7
(22) Date of filing: 10.08.2004
(51) Int. Cl.: A61K 36/185, A61K 6/00, A61P 17/08

(54) **COMPOSITION COMPRISING THE EXTRACT OF ACTINIDIA ARGUTA FOR PREVENTING AND TREATING ALOPECIA AND SEBORRHEIC SKIN DISORDERS**
ZUSAMMENSETZUNG AUS DEM EXTRAKT VON ACTINIDIA ARGUTA ZUR PRÄVENTION UND BEHANDLUNG VON ALOPEZIE UND SEBORRHÖISCHEN HAUTERKRANKUNGEN
COMPOSITION COMPRENANT L'EXTRAIT DE ACTINIDIA ARGUTA SERVANT À PRÉVENIR ET À TRAITER ALOPECIE ET LES TROUBLES SÉBORRHÉIQUES DE LA PEAU

(43) Date of publication of application: 13.06.2007
(73) Proprietor: Helixir Co., Ltd., Shillim 9-dong, Kwanak-Gu Seoul 151-742 (KR)
(72) Inventor: KIM, Bongcheol, Gyeonggi-do 427-802 (KR); EO, Hae Kwan, Seoul 135-280 (KR); LEE, Hwa-Jun, Gwonseon-gu, Gyeonggi-do 441-831 (KR); JIN, Mirim, Gangnam-gu Seoul 138-542 (KR); JUNG, Hyung-Jin, Sung-dong-Gu Seoul 133-762 (KR); KIM, Sunyoung, Gyunggi-do 487-821 (KR)
(74) Representative: Grünecker, Kinkeldey, Stockmair & Schwanhäusser
(86) International application number: PCT/KR2004/002010
(87) International publication number: WO 2006/016728

(56) References cited:
- EP-A2- 0 569 667
- DE-A1- 4 129 066
- DE-A1- 19 758 090
- JP-A- 2 202 808
- JP-A- 5 000 962
- JP-A- 10 182 475
- JP-A- 2002 145 754
- JP-A- 2002 173 416
- KR-A- 2004 097 716

## Description

### Technical Field

The present disclosure, comprising the invention, relates to a composition comprising an extract of *Actinidia arguta* having preventing and treating activity of baldness disorder and seborrheic skin disease.

### Background Art

The present invention relates to a composition comprising hardy kiwifruit extract having preventing and treating activity of baldness disease and seborrheic skin disease , and especially, a composition comprising the crude extracts, non-polar solvent soluble extracts, or purified fraction of kiwifruit extract for the prevention and treatment of baldness disease and seborrheic skin disease.

Baldness syndrome is frequently occurred in middle-aged male and classified into two types of baldness. One is male pattern baldness alternatively named as an alopecia seniles or androgenic alopecia generally occurred in the forehead or calvaria of middlc-agcd male, and another is alopecia areata which shows relatively concrete border line of alopecia and frequently being occurred in juvenile person. The syndrome is mainly occurred in male and even in female (M. lnaba et al; Androgenetic alopecia, Springer-Berlag, Tokyo, Japan, 1996).

There has been reported that alopecia is originated from genetic factor, the progress of aging, drug abuse, radioactive treatment and the stress occurred after serious illness etc. It has been known that main factors of alopecia are over-production of androgenic hormone, blood circulation problem in scalp and vitamin deficiency essential to hair metabolism in biochemical and physiological aspects and the main factor of male pattern baldness relates to androgen and steroid hormone, in particular, 5-alpha-reductase converting testosterone to 5-alpha-dihydrotestosterone (DHT) and being distributed in sebaceous gland, keratinocytes of hair follicle, dermal papilla cell, sweat gland, root sheath of the scalp hair follicle etc and aromatase converting androgen to female hormone such as estradiol. The etiological origin of alopecia areata is known to be the disorder of autoimmune system, especially the lymphocyte CD4+ disorder in melanocyte, keratinocyte, dermal papilla and vascular endothelium (J. Shapiro et al; Therapeutic agents, Dermatol. Clin., 16(2) pp341-356, 1998).

Various conventional drugs for the treatment of alopecia have been developed and used till now. For example, finasteride, an oral 5-alpha-reductase inhibitor, has been available in the market, however it has several disadvantages such as repetitive recurrence in case of the cease of administration, limit to the use in pregnant women, and sexual dysfunction due to long-term administration; minoxidil, one of available drug in the market, has been used to treat male pattern baldness together with other agents for example, cell division accelerator, vasodilator, capillary formation promotor, immuno-suppressor, cAMP modulator, EGF(epithelial cell growth factor) and cell proliferation modulator, however it has also adverse effect such as repetitive recurrence and rapid blood pressure lowering effect in case of the cease of administration too; ACTH (adrenocorticotrophin) as a representative immuno-modulating agent acting on lymphocyte to suppress hyperactive immune system has been administrated to treat alopecia areata by a form of ointment or injection, however it has also several disadvantage such as recurrence or adverse effect, which cause to difficulty in long term administration. Besides above described drugs, cyproterone actate, spironolactone, estrogen and so on have been reported to treat alopecia (J. Shapiro et al, Hair regrowth, Therapeutic agents, Dermatol. Clin. 16(2), pp341-356, 1998)

As described above, most of conventional drugs do not provide with enough efficacy to accomplish satisfactory activity and have several advantages such as a recurrence or adverse effect, therefore there have been lots of approach to obtain new drug which provides with satisfactory effect and reduced adverse effect till now.

Meanwhile, there has been lots of concentrated effort to investigate effective drug from natural resource to treat and prevent alopecia disease till now.

As an alternative approach, there have been several reports on the composition comprising an extract of crude drug having hair growth stimulating effect, for example, plant extract belonged to Orchidaceae disclosed in Korea Patent Registration No. 015667, vitex seed and bear fat in Korea Patent Registration No. 0161338, an extract of *Rhododendron fauriae* in Korea Patent Publication No. 91-106, pine leaf extract in Korea Patent Publication No. 96-40346, an extract of crude drug consisting of torilla seed, pine leaf and silkworm moth in Korea Patent Publication No. 90-2757, an extract of crude drug consisting of *Acanthopanax sessiliflorus, Astragalus membranceus, Polygonum multiflorum, Dioscorea nipponica* and *Fagopyrum esculentum* in Korea Patent Publication No. 2000-24499, an extract of crude drug consisting of *Pinellia ternata, Eugenia Caryophyllata, Rubus coreanus, Zanthoxylum piperatum, Vitex rotundifolia, Salvia milliorrhiza,* and *Thujae semen* in Korea Patent Registration No. 259037, the fat obtained by heating silkworm and their byproduct, at 200 °C for 15 mins, an extract of crude drug consisting of *Panax ginseng, Angelica sinensis, Astragalus membranceus, Polygonum multiflorum, Juglans sinensis, Rubus coreanus, Rehmannia glustinosa, Psoralea corylifolia, Pinus densiflora, Vitex rotundifolia, Carthamus tinclorius* and *Ligustrum lucidum* in Korea Patent Publication No. 90-13934, an extract of crude drug consisting of silkworm moth, silkworm eggs, silkworm pupa, black sesame seeds, sesame seeds, brown seaweed, and walnuts in Korea Patent Publication No. 90-5952, mixed powder of mung beans, soy bean, perilla seeds, sesame seeds, walnuts, laver, sea mussel, oyster, anchovy, brown seaweed, and yeast concentrates in Korea Patent Registration No. 260673, an extract of fruits, root, branch, leaf, flowers of *Lycium chinensis,* and corn silk in Korea Patent Publication No. 2000-36534 etc. However, the hair growing effect of above described composition disclosed therein have not been fully supported or identified in any of disclosure herein.

*Actinidia arguta, A. polygama* and *A. kolomikta* belonged to *Actinidiaceae,* are distributed in Siberia, the northern area of China , North and South Korea. More than 30 species belonged to *Actinidiaceae* have been reported. Among them, the fruit of *Actinidia chinensis* or A. *delicious* have been named as a kiwi and *Actinidia arguta,* and other identical genus fruit have been used as materials of Chinese medicine named as 'mihudo', to treat liver disease, gastrointestinal disease and urogenital lithiasis without toxicity (Chung B. S. and Shin M. K.; HvangyakDaesacheon, Youngrimsa., pp386-388, 1998).

Meanwhile, there have been several applications regarding on the activity of Actinidia fruit to treat or prevent various medicinal diseases till now.

For example, Korea Patent Registration No. 344147 discloses health beverage containing an exudates of Actinidia fruit as a main component and the preparation thereof; Korea Patent Registration No. 134024 discloses health beverage containing an fruit extract of *Actinidia chinensis* or *A. delicious* as a main component and the preparation thereof; Korea Patent Publication No. 1996-37061 discloses a h epato-protective agent and anti-cancer agent comprising an extract of Actinidia fruit as a main components; Korea Patent Publication No. 2002-78467 discloses health beverage containing an extract of crude drugs consisting of *Actinidia arguta ,Hovenia dulcis, Lycium chinensis, Schizandra chinensis, Artemis capillaries, Pueraria thunbergiana, Glycyrrhiza uralensis, Lagenaria siceraria, Aralia elata, Polygala tenuifolia, Angelica sinensis, Atractylodes macrocephala, Garclenia jasminoides, Asiragalus membranaceus, Carthamus tinctorius and Cordyceps militaris* for the improvement of liver function.

However, there has been not reported or disclosed about treating or preventing activity of hardy kiwifruit extract on alopecia and seborrheic skin disease in any of above literatures.

To investigate the treating effect and preventing effect of heady kiwifruit extract on alopecia and seborrheic skin disease, the inventors of present invention have intensively carried out *in vivo* and *in vitro* experiment concerning the inhibition effect on the reproduction of DHT (dihydrotestosterone) together with clinical experiment concerning on the hair growth stimulation and seborrheic skin disease. As a result of the investigation, the inventors finally completed the present invention by confirming that the hardy kiwifruit extract reduced blood DHT level, and inhibited the falling out of hair and seborrheic skin disease.

### Disclosure

The present invention provides a pharmaceutical composition comprising a crude or purified extract of hardy kiwifruit as an active ingredient for use in the treatment and prevention of alopecia, seborrheir dermatitis, keratogenous skin disorder and pruritic scalp, wherein the crude or purified extract is soluble in water, lower alcohol such as methanol, ethanol and butanol or mixtures thereof or in ethyl acetate.

The present invention provides a use of said extracts of hardy kiwifruit for the preparation of pharmaceutical composition for treating and preventing of the above diseases in human and mammal.

The present invention also provides a health care food a food additive, a feed additive and a feed composition comprising said extract for treating and prevention of the above diseases.

Above described alopecia disorders herein comprise alopecia seniles, alopecia areata.

The term 'crude extract' defined herein means 'the extract' soluble in water, lower alcohol such as methanol, ethanol or butanol and the mixture thereof, preferably, organic solvent mixture mixed with water and ethanol.

Preferably, above described crude extract herein comprise the extract obtained by the step consisting of: extracting hardy kiwi with water, lower alcohol such as methanol, ethanol or butanol and the mixture thereof, preferably, organic solvent mixture mixed with water and ethanol; filtrating and concentrating the filtrate.

Above described ethyl acetate soluble extract herein comprises the extract obtained by the step consisting of: extracting hardy kiwi with ethylactate; filtrating and concentrating the filtrate.

Above hardy kiwifruit may comprises *Actinidia arguta, A. kolomikta, A. polygama* or and the same genus plant and may use the fruit, stem, root thereof.

The pharmaceutical composition of the present invention can contain about 0.01 - 50 % by weight of the above extract based on the total weight of the compositions.

The health care food of the present invention comprises above extracts as 0.01 to 95 %, 80 % by weight based on the total weight of the composition.

Above health care food can be contained in health food, health beverage etc, and may be used as powder, granule, tablet, chewing tablet, capsule, beverage etc.

An inventive extract of hardy kiwifruit can be prepared in detail by following procedures,

The inventive crude extract of hardy kiwifruit can be prepared by follows; hardy kiwifruit is dried, cut, crushed and mixed with 5 to 25-fold, preferably, approximately 10 fold volume of distilled water, lower alcohols such as methanol, ethanol, butanol and the like, or the mixtures thereof, preferably methanol; the solution is treated with hot water at the temperature ranging from 20 to 100 °C, preferably from 60 to 100 °C, for the period ranging from 1 to 24 hours with extraction method by the extraction with hot water, cold water, reflux extraction, or ultra-sonication extraction with 1 to 5 times, preferably 2 to 3 times, consecutively; the residue is filtered to obtain the supernatant to be concentrated with rotary evaporator, at the temperature ranging from 20 to 100 °C, preferably from 50 to 70 °C and then dried by vacuum freeze-drying, hot air-drying or spray drying to obtain dried crude extract powder of hardy kiwifruit which can be soluble in water, lower alcohols, or the mixtures thereof.

Additionally, polar solvent soluble and non-polar solvent soluble extract of present invention can be prepared by following procedure; the crude extract prepared by above step, is suspended in water, and then is mixed with 1 to 100-fold, preferably, 1 to 5-fold volume of non polar solvent such as ethyl acetate, chloroform, hexane and the like; the non-polar solvent soluble layer is collected to obtain non-polar solvent soluble extract of the present invention and remaining polar solvent soluble layer is collected to obtain polar solvent soluble extract of the present invention which is soluble in water, lower alcohols, or the mixtures thereof. Also, above described procedures may be modified or subjected to further step to fractionate or isolate more potent fractions or compounds by conventional procedure well- known in the art, i.e., the procedure disclosed in the literature (Harbome J. B. Phytochemical methods: A guide to modern techniques of plant analysis, 3rd Ed. pp6-7, 1998).

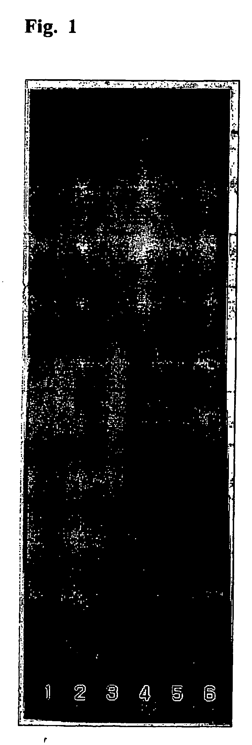

For example, the further purified fractions of hardy kiwi extract of the present invention can be prepared by subjecting ethyl acetate soluble extract prepared by aforementioned step to column chromatographic process such as Silicagel column chromatography, TLC (Thin layer chromatography) or Sephadex column chromatography, preferably, Silicagel column chromatography eluting with eluting solvent mixed with chloroform: methanol: water in order to increasing their polarity in stepwise manner, more preferably, starting with mixed ratio of 100:10:1(W/W/W) and ending with 20:10:0.5(W/W/W) to obtain further purified extract, in particular, the purified extracts having TLC spectra as shown in Fig. 1 to 3.

The term 'purified extract' defined herein means any of 'fractions' obtained by column chromatographic purification having more potent activity than that of polar or non-polar solvent soluble extract, preferably, the purified extracts having TLC spectra as shown in Fg. 1 to 3.

To investigate the inhibiting activity of the crude extracts, ethyl acetate soluble extract and purified extract of hardy kiwifruit extract prepared by above procedure on the claimed diseases *in vivo* and *in vitro* experiments concerning the inhibition effect on the reproduction of DHT (dihydrotestosterone) in mouse blood and on the formation of mouse hair root together with clinical experiment concerning on the hair growth stimulation and seborrheic skin disease were carried out. As a result of the investigation, the inventors confirmed that orally administrated hardy kiwifruit extract reduced blood DHT level, promoted the formation of hair root in mouse model experiment, and inhibited the falling out of hair and improved seborrheic skin disease of volunteers such as keratogenous disorder dermatitis skin seborrheic etc.

The pharmaceutical composition may additionally comprise conventional carrier, adjuvant or diluents in accordance with conventional using method well known in the art.

The pharmaceutical composition according to the present invention can be provided as a composition containing pharmaceutically acceptable carriers, adjuvants or diluents, e.g., lactose, dextrose, sucrose, sorbitol, mannitol, xylitol, erythritol, maltitol, starches, acacia rubber, alginate, gelatin, calcium phosphate, calcium silicate, cellulose, methyl cellulose, polyvinyl pyrrolidone, water, methylhydroxy benzoate, propylhydroxy benzoate, talc, magnesium stearate and mineral oil. The formulations may additionally include fillers, anti-agglutinating agents, lubricating agents, wetting agents, flavoring agents, emulsifiers, preservatives and the like. The compositions of the invention may be formulated so as to provide quick, sustained or delayed release of the active ingredient after their administration to a patient by employing any of the procedures well known in the art.

For example, the compositions of the present invention can be dissolved in oils, propylene glycol or other solvents that are commonly used to produce an injection. Suitable examples of the carriers include physiological saline, polyethylene glycol, ethanol, vegetable oils, isopropyl myristate, etc., but are not limited to them. For topical administration, the compounds of the present invention can be formulated in the form of ointments and creams.

Pharmaceutical formulations containing present composition may be prepared in any form, such as oral dosage form (powder, tablet, capsule, soft capsule, aqueous medicine, syrup, elixirs pill, powder, sachet, granule), or topical preparation (cream, ointment, lotion, gel, balm, patch, paste, spray solution, aerosol and the like), or injectable preparation (solution, suspension, emulsion).

The composition of the present invention in pharmaceutical dosage forms may be used in the form of their pharmaceutically acceptable salts, and also may be used alone or in appropriate association, as well as in combination with other pharmaceutically active compounds.

The desirable dose of the inventive extract or composition varies depending on the condition and the weight of the subject, severity, drug form, route and period of administration, and may be chosen by those skilled in the art. However, in order to obtain desirable effects, it is generally recommended to administer at the amount ranging 10 g/kg, preferably, 1 to 3 g/kg by weight/day of the inventive extract or compounds of the present invention. The dose may be administered in single or divided into several times per day.

The pharmaceutical composition of present invention can be administered to a subject animal such as mammals (rat, mouse, domestic animals or human) via various routes. All modes of administration are contemplated, for example, administration can be made orally, rectally or by intravenous, intramuscular, subcutaneous, intracutaneous, intrathecal, epidural or intracerebroventricular injection.

Also, the present invention provide a health care food (beverage) for prevention and treatment of the above diseases adding the hardy kiwifruit 0.01 to 20 % by weight, amino acids 0.001 to 5 % by weight, vitamins 0.001 to 2 % by weight, sugars 0.001 to 20 % by weight, organic acids 0.001 to 10 % by weight, sweetener and flavors of proper amount.

To develop for healt care food, examples of addable food comprising above extracts of the present invention are e.g., various food, beverage, gum, vitamin complex, health improving food and the like, and can be used as power, granule, tablet, chewing tablet, capsule or beverage etc.

Also, the extract of present invention may be added to child and infant food, such as modified milk powder, modified milk powder for growth period, modified food for growth period.

The amount or above described extract in health care may generally range from about 0.01 w/w% to 80 w/w % of total weight of feed. the health care food composition and 1 to :i0 g, preferably 3 to 10 g on the ratio of 100 ml of the health beverage composition.

Providing that the health everage care food, like composition of present invention contains above described extract as an essential component in the indicated ratio, there is no particular limitation on the other liquid components, wherein the other component can be various deodorant or natural carbohydrate etc such as conventional beverage. Examples of aforementioned natural carbohydrate are monosaccharide such as glucose, fructose etc; disaccharide such as maltose, sucrose etc; conventional sugar such as dextrin, cyclodextrin; and sugar alcohol such as xylitol, and erythritol etc. As the other deodorant than aforementioned ones, natural deodorant such as taumatin, stevia extract such as levaudioside A, glycyrrhizin et al., and synthetic deodorant such as saccharin, aspart am et al., may be useful favorably. The amount of above described natural carbohydrate is generally ranges from about 1 to 20 g, preferably 5 to 12 g in the ratio of 100 ml of present beverage composition.

The other components than aforementioned composition are various nutrients, a vitamin, a mineral or an electrolyte, synthetic flavoring agent, a coloring agent and improving agent in case of cheese chocolate et al., pectic acid and the salt thereof, alginic acid and the salt thereof, organic acid, protective colloidal adhesive, pH controlling agent, stabilizer, a preservative, glycerin, alcohols, carbonizing agent used in carbonate beverage et al. The other component than aforementioned ones may be fruit juice for preparing natural fruit juice, fruit juice beverage and vegetable beverage, wherein the component can be used independently or in combination. The ratio of the components is not so important but is generally range from about 0 to 20 w/w % per 100 w/w % present composition. Examples of addable food comprising aforementioned extract therein are various food, beverage, gum, vitamin complex, health improving food and the like.

The inventive composition may additionally comprise one or more than one of organic acid, such as citric acid, fumaric acid, adipic acid, lactic acid, manic acid; phosphate, such as phosphate, sodium phosphate, potassium phosphate, acid pyrophosphate, polyphosphate; natural anti-oxidants, such as polyphenol, catechin, alpha-tooopherol, rosemary extracts, vitamin C, green tea extract, licorice root extract, chitosan, tannic acid, phytic acid etc.

The above extract of the hardy kiwifruit may be 20 to 90 % high concentrated liquid, power, or granule.

Similarly, the above extract of the hardy kiwifruit can comprise additionally one or more than one of lactose, casein, dextrose, glucose, sucrose and sorbitol.

Also, in the present invention, there is also provided a using method of the food additives such as sterilizer, spice, seasoning, various nutrients, vitamin, a mineral or an electrolyte, synthetic flavoring agent, a coloring agent and improving agent in case of cheese chocolate et al., pectic acid and the salt thereof, alginic acid and the salt thereof, organic acid, protective colloidal adhesive, pH controlling agent, stabilizer, a preservative, glycerin, alcohols, carbonizing agent used in carbonate beverage et al, or as essential component of food materials.

Wherein the food additives can be added to food by deposition, spray, or mixing the ratio of the additives is not so important but is generally range from about 0.01 to 20 w/w % per 100 w/w % present compositions. Examples of addable food comprising aforementioned extract therein are.

Wherein the food additives can be added to one or one over food such as fruits, vegetables, food dehydrated foods or cutting products such as fruits, vegetables; fruit juice, vegetable juices or the mixture juices thereof; drinks containing acid-beverage; confectionaries such as cookie, candy, caramel, gum; breads; ice creams, teas, fermented milk such as yogurt; dairy product, spices, alcoholic beverages, cans, in-bottles, noodles, processed livestock products, processed marine products, fermented food, beans food, cereals food, processed meats, licorices or hubs.

In accordance with another aspect of the present invention, there are provided a feed or feed additive essentially comprising said extract prepared by above preparation method for prevention and improvement baldness disorder and seborrheic skin disease

Above food additives of the present invention can be mixed with mixing amount of 5 to 100 g per 1 kg by weight based on the total dried weight of the feed.

Furthermore, the present invention provides a feed composition comprising above feed additives.

The pharmaceutical composition of the present invention may be formulated in any form such as skin, lotion, cream, essence, toner, emulsion, pack, soup, shampoo, rinse, cleanser, body washing solution, washing solution, treatment, gel, balm, spray solution and the like.

Such a may comprise additional additives selected from the group consisting of water soluble vitamin, lipid soluble vitamin, peptide polymer, polysaccharide polymer, sphingolipid and sea-weed extract.

Preferable water soluble vitamins are any one which can be mixed with cosmetic, however, various vitamin such as vitamin B1, B2, B6, pyridoxine, pyridoxine HCl, vitamin B12, pantothenic acid, nicotinic acid, nicotinamide, folic acid, vitamin C, vitamin H etc, their salt thereof such as thiamin HCl salt, ascorbic acid Na salt etc or their derivatives thereof such as ascorbic acid-2-phosphonic acid Na salt, ascorbic acid-2-phosphonic acid Mg salt are preferable and those can be obtained by conventional method such as microbial conversion method, purification method from the microbial cultivates, enzymatic method or chemical synthetic method.

Preferable lipid soluble vitamins are any one which can be mixed with cosmetic, however, various vitamin such as vitamin A, D2, D3, E (dl-alpha-tocopherol, d-alpha-tocopherol, d-delta-tocopherol) and their derivatives such as palmitic acid ascorbate, stearic acid ascorbate, dipalmitic acid ascorbate, acetic acid- dl-alpha-tocopherol, nicotinic acid dl-alpha-tocopherol vitamin E, dl-pantothenyl alcohol, D-pantothenyl alcohol, pantothenyl ethylether etc. containing the lipid soluble vitamin used in examples of present invention are preferable and those can be obtained by conventional method such as microbial conversion method, purification method from the microbial cultivates, enzymatic method or chemical synthetic method.

Preferable peptide polymers are any one which can be mixed with cosmetic, however, collagen, hydrolysable collagen, gelatin, elastin, hydrolysable gelatin, keratin etc. containing the peptide polymer used in examples of present invention are preferable.

Preferable polysaccharide polymers are any one which can be mixed with cosmetic, however, hydroxy ethyl cellulose, xanthin gum, hyaluronic acid Na, chondroitin sulfate or their salt (Na salt etc) and the like are preferable. For example, chondroitin sulfate or the salt thereof etc can be used by being purified from mammal or fishes ordinarily.

Preferable sphingolipid are any one, which can be mixed with cosmetic, however, ceramide, pit-sphingosin, sphingo-lipopolysaccharide and the like are preferable. Sphingo-lipid can be obtained by being purified from mammal, fish, shellfish, yeast or plant etc in conventional method.

Preferable seaweed extract is any one which can be mixed with cosmetic, however, the extract of brown algae, red algae, green algae and the like or the purified carrageenan, alginic acid, arginic acid Na, K isolated therefrom are preferable. Algae extract can be obtained by being purified from seaweed in conventional method.

Other ingredients which may be added to the composition, may comprises oil ingredient, humectants, emollients, surface active agents, organic or inorganic dye, organic powder, ultraviolet ray absorbing agent, preservatives, antiseptics, antioxidants, plant extract, pH controlled, alcohols, pigments, perfumes, refrigerants, blood circulator, antihidrotic, distilled water etc.

Preferable oil ingredients may comprise ester oil, hydrocarbon oil, silicone oil, fluoride oil, animal oil, plant oil and so on.

Preferable ester oil described above may comprise glyceryl tri-2-ethyl hexanoic acid, cetyl 2-ethyl hexanoic acid, isopropyl myristic acid, butyl myristic acid, isopropyl palmitic acid, ethyl stearic acid, octyl palmitic acid, isocetyl isostearic acid, butyl stearic acid, ethyl linoleic acid, isopropyl linoleic acid, ethyl oleic acid, isocetyl myristic acid, isostearyl myristic acid, isostearyl palmitic acid, octyldodecyl myristic acid, isocetyl isostearic acid, diethyl sebasic acid, isopropyl adipic acid, isoalkyl neopetanoic acid, glyceryl tri(capryl, capric acid), trimethylopropane tri-2-ethyl hexanoic acid, trimethylopropane triisostearic acid, pentaerythritol tetra-2 ethyl hexanoic acid, cetyl caprylic acid, decyl lauric acid, hexyl lauric acid, decyl myristic acid, myristyl myristic acid, cetyl myristic acid, stearyl stearic acid, decyl oleic acid, cetyl licinoleic acid, isostearyl lauric acid, isotridecyl myristic acid, isocetyl palmitic acid, octyl stearic acid, isocetyl stearic acid, isodecyl oleic acid, octyldodecyl oleic acid, octyldodecyl linoleic acid, isopropyl isostearic add, cetostearyl 2-ethyl hexanoic acid, stearyl 2-ethyl hexanoic acid, hexyl isostearic acid, ethylene glycol dioctanoic acid, ethylene glycol dioleic acid, propylene glycol dicapric acid, propylene glycol di(capryl, capric acid), propylene glycol dicaprylic acid, neopentylglycol dicapric acid, neopentylglycol dioctanoic acid, glyceryl tricaprylic acid, glyceryl triundecylic acid, glyceryl triisopalmitic acid, glyceryl triisostearic acid, octyldodecyl neopentanoic acid, isostearyl octanoic acid, octyl isononanoic acid, hexyldecyl neodecanoic acid, octyldodecyl neodecanoic acid, isocetyl isostearic acid, isostearyl isostearic acid, octyldecyl isostearic acid, polyglycerin oleanoic acid ester, polyglycerin isostearic acid ester, triisocetyl citric acid, triisoalkyl citric acid, triisooctyl citric acid, lauryl lactic acid, myristyl lactic acid, cetyl lactic acid, octyldecyl lactic acid, triethyl citric acid, acetyltriethyl citric acid, acetyl tributyl citric acid, trioctyl citric acid, diisostearyl maleic acid, di 2-ethylhexyl hydroxy stearic acid, 2-ethyl hexyl succinic acid, diisobutyl adipic acid, diisopropyl sebasinic acid, dioctyl sebacinic acid, cholesteryl stearic acid, cholesteryl isostearic acid, cholesteryl hydroxy stearic acid, cholesteryl hydroxy stearic acid, cholesteryl oleic acid, dihydrocholesteryl oleic acid, pitsteryl isostearic acid, pitsteryl oleic acid, isocetyl 12-stealoyl hydroxy stearic acid, stearyl 12-stealoyl hydroxy stearic acid, isostearyl 12-stealoyl hydroxy stearic acid.

Preferable hydrocarbon oil described above may comprise squalene, liquid paraffin, alpha-olefin oligomer, isoparaffin, ceresin, paraffin, liquid isoparaffin, polybuden, microcrystalline wax, vaselin and the like.

Preferable silicone oil may comprise polymethylsilicone, methylphenylsilicone, methylcyclopolysiloxane, octamethylpolysiloxane, decamethylpolysiloxane, dodecamethylcyclosiloxane, dimethyl siloxane-methyl cetyloxysiloxan copolymer, dimethyl siloxane-methyl stealoxysiloxane copolymer, alkyl modified silicone oil, amino modified silicone oil and the like.

Preferable fluoride oil can comprise perfluoropolyether and the like.

Preferable animal or plant oil can comprise avocado oil, almond oil, olive oil, sesame oil, rice husk oil, safflower oil, soy-bean oil, corn oil, rape oil, amygdalin oil, palm kernel oil, palm oil, pimaja oil, sunflower oil, fruite seed oil, cotton seed oil, coconut palm oil cucui nut oil, wheat embryo bud oil, rice embryo bud oil, sia butter, evening-primrose oil, marker daymia nut oil, medo home oil, egg yolk oil, lanolin, hempseed oil, mink oil, orange ruppy oil, hohoba oil, camawa wax, liquid lanolin, solid pimaja wax and the like.

Preferable humectants can comprise water-soluble low molecular humectants, lipophilic low molecular humectants, water-soluble polymer and lipid soluble polymer.

Specifically, preferable water soluble low molecular humectants can comprise cerin, glutamine, sorbitol, mannitol, pyrrolidone-carboxylic acid Na, glycerin, propylene glycol, 1,3-butylene glycol, ethylene glycol, polyethylene glycol (polymerization index >2), polypropylene glycol (polymerization index >2), lactic acid, lactate salt and the like.

Preferable lipid soluble low molecular humectants can comprise cholesterol, cholesteryl ester and the like.

Preferable water-soluble polymer can comprise carboxy vinyl polymer, poly asparaginic acid salt, tragacanth, xanthin gum, HMC (hydroxy methyl celluose), HEC (hydroxy ethyl celluose), HPC (hydroxy propyl celluose), carboxymethylcellulose, water-soluble chitin, chitosan, dextrin and the like.

Preferable lipid soluble polymer can comprise polyvinylpyrrolidone-eicocene copolymer, polyvinylpyrrolidone-hexadecene copolymer, nitrocellulose, dextrin fatty acid ester, silicone polymer and the like.

Preferable emollients can comprise long chain acyl glutamic acid cholesteryl ester, cholesteryl hydroxy stearic acid, 12-hydroxy stearic acid, rogic acid, lanolin fatty acid cholesteryl ester and the like.

Preferable surface-active agent can comprise nonionic surfactants, anionic surfactants, cationic surfactants, ambivalent surfactants and the like.

Specifically, preferable non-ionic surfactants can comprise self-emulsified monostearic acid glycerin, propylene glycol fatty acid ester, glycerin fatty acid ester, polyglycerin fatty acid ester, sorbitan fatty acid ester, polyoxyethylene (POE) sorbitan fatty acid ester, POE sorbitan fatty acid ester, POE glycerin fatty acid ester, POE alkyl ether, POE fatty acid ester, POE solid pimaja oil, POE pimaja oil, POE-POP polymer, POE-POP alkyl ether, polyether modified silicone, lauric acid alkanol amide, alkyl amine oxide, hydrogen addition soybean phospholipid and the like.

Preferable anionic surfactants can comprise fatty acid soap, a -acyl sulfonic acid salt, alkyl sulfonic acid salt, alkyl ally sulfonic acid, alkyl naphthalene sulfonic acid salt, alkyl sulfonic acid salt, POE alkylether sulfate salt, alkyl amide sulfate salt, alkyl phosphate salt, POE alkyl phosphate salt, alkylamide phospahate salt, alkyloylalkyl taurine salt, N-acyl-amino acid salt, POE alkyl ether carboxylic acid salt, alkyl sulfo succinic aid salt, alkyl sulfo-acetic acid salt, acylated hydrolysable collagen peptide salt, perfluoro alkyl phosphate ester and the like.

Preferable cationic surfactant can comprise alkyl trimethyl ammonium chloride, stearyl trimethyl ammonium chloride, stearyl trimethyl ammonium bromide, setostearyltrimethyl ammonium chloride, distearyl dimethyl ammonium chloride, stearyl dimethyl benzyl ammonium chloride, vehenyltrimethyl ammonium bromide, benzalkonium chloride, diethylamino ethyl amide stearic acid, dimethylaminopropyl amide stearic acid, lanolin derivatives quaternary ammonium and the like.

Preferable ambivalent surfactants can comprise carboxy betaine type, amide betaine type, hydroxy sulfo betaine type, phosphpbetaine type, aminocarboxylic acid, imidazoline derivatives type, amide amine type and the like.

Preferable organic and inorganic dyes can comprise silicic acid, anhydrous silicic acid, magnesium silicic acid, talc, ceracyte, mica, caolin, bengala, clay, bentonite, titan film mica, oxy chlorine bismuth, zirconium oxide, magnesium oxide, zinc oxide, titan oxide, aluminium oxide, calcium sulfate, barium sulfate, magnesium sulfate, calcium carbonate, magnesium carbonate, ferrous oxide, chromium oxide, chromium hydroxide, calamine, carbon black and their complex thereof as an inorganic dyes; polyamide, polyester, polypropylene, polystyrene, polyurethane, vinyl resin, urea resin, phenol resin, fluoride resin, silicone resin, acryl resin, melamine resin, epoxy resin, polycarbonate resin, divinyl benzene-styrene copolymer, silk powder, cellulose, CI pigment yellow, CI pigment orange as an organic dyes; and their complex etc.

Preferable organic powder can comprise metal soap such as calcium stearate; alkyl phosphonate metal salt such as sodium zinc cetylic acid, zinc laurylic acid, calcium laurylic acid; acylamino acid polyvalent metal salt such as calcium N-lauroyl-beta-alanine, zinc N-lauroyl-beta-alanine, calcium N-lauroyl-glycine etc.; amide sulfonic acid polyvalent metal salt such as calcium N-lauroyl-taurine, calcium N-palmitoyl-taurine; N-acyl basic amino acid such as N epsilon-lauroyl-L-lysine, N epsilon-palmitoyl-lysine, N alpha-palmitoyl ornitine, N alpha-lauroly arginine, hardened lanolin fatty acid acyl arginine and the like; N-acylpolypeptide such as N-lauroylglycyl glycine; alpha-amino fatty acid such as alpha-amino caprylic acid, alpha-amino lauric acid and the like; polyethylene, polypropylene, nylon, polymethylmetacrylate, polystyrene, divinylbenzene-styrene copolymer, ethylene tetrafluoride and so on.

Preferable ultraviolet absorbing agents can comprise paraaminobenzoic acid, paraamonoethyl benzoate, paraamino amyl benzoate, paraamino octyl benzoate, ethyleneglycol salicylate, phenyl salicylate, octyl salicylate, benzyl salicylate, butylphenyl salicylate, homomentyl salicylate, benzyl cinnamic acid, paramethoxy 2-ethoxy ethyl cinnamic acid, paramethoxy octyl cinnamic acid, diparamethoxy mono-2-ethylhexane glyceryl dynamic acid, paramethoxy isopropyl cinnamic acid, diisopropyl-diisopropyl cinnamate ester mixture, urokanic acid, ethyl urokanic acid, hydroxy methoxy benzophenone, hydroxymethoxy benzophenone sulfonic acid and their salt, dihydroxy methoxy benzophenone, dihydroxy methoxy benzophenone disulfonate Na, dihydroxy benzophenone, tetrahydroxybenzophenone, 4- *tert -* butyl-4'-methoxydibenzoylmethane, 2,4,6-trianilino-*p*-(carbo-2'-ethyl hexyl-1'-oxy)-1,3,5-triazine, 2-(2-hydroxy-5-methylphenyl) benzotriazole and the like.

Preferable preservatives can comprise hinokitiol, trichloric acid, trichlorohydroxy-diphenylether, chlorohexidine glucuronate, phenoxyethanol, resorcine, isopropylmethylphenol, azulene, salicylic acid, zinc pilithione, bezalconium HCl, photosensitizer 301, mononitroguaiacol Na, undecylenic acid etc.

Preferable antioxidants can comprise butylhydroxyanisole, propyl gallate, ellisorbate and the like.

Preferable pH controller can comprise citric acid, sodium citrate, malic acid, sodium malate, fumaric acid, sodium fumaric acid, succinic acid, sodium succinic acid, sodium hydroxide, sodium hydrogen phosphate and the like.

Preferable alcohol can comprise cetyl alcohol etc.

Furthermore, other ingredient addable to above described component and the amount thereof is not limited within the scope of the purpose and effect of the present invention, however, it is preferable that the amount of the other ingredients ranges from 0.01 to 5%, more preferably, 0.01 to 3% in that of total composition.

The composition of the present invention can be modified as a solution, emulsion, cohesive mixture etc.

Above described ingredients such as water-soluble vitamin, lipid soluble vitamin, peptide polymer, polysaccharide polymer, sphingolipid, sea weed extract and addable ingredients which can be added other than above described ingredients if necessary, can be obtained by conventional methods disclosed in the literature (Matsumoto Mithio, Manual for the development of transdermal applied preparation. Seisi Press, 1 st Ed., 1985).

The extracte of the present invention have no toxicity and adverse effect therefore; they can be used with safe.

### Description Of Drawings

Fig. 1 shows TLC photograph of the extracts and fractions of hardy kiwifruit;

Fig. 2 shows 2D-TLC photograph of [1] sub-fraction;

Fig. 3 shows 2D-TLC photograph of [2] sub-fraction;

Fig. 4 presents the lowering effect of hot-water extract of hardy kiwi on the blood DHT concentration in mouse;

Fig. 5 presents the lowering effect of non-polar solvent soluble extract of hardy kiwi on the blood DHT concentration in mouse;

Fig. 6 presents the lowering effect of purified extract of hardy kiwi on the blood DHT concentration in mouse;

Fig. 7 and 8 show the photographs of mouse hair grown in its back area treated with control and the kiwi extract for two weeks orally respectively.

### Examples

**Example 1. Preparation of hardy kiwifruit extract**

**1-1. Preparation of water extract of hardy kiwifruit**

100g of dried hardy fruit and dried stem of hardy kiwifruit (*Actinidia arguta*), dried fruit of *A. kolomikta* and *A. polygama* purchased from Kyung-dong Market located in Seoul was crushed, mixed with 1 L of distilled water and subjected to reflux extraction for 3 hrs at 90 ∼ 95 °C with three times and the extract was filtered with filter paper, concentrated using by rotary evaporator (N-1000, Eyela Co. Japan) at 55 - 65 °C under reduced pressure and dried with freezing dryer to obtain 15.6 g of dried fruit extracts, 10.4 g of dried stem extract of kiwifruit (*Actinidia arguta*), 16.2 g and 17.0g of dried fruit extract of *A*. *kolomikta,* and *A. polygama* respectively The dried powder was dissolved in distilled water (100 mg/ml).

**1-2. Preparation of water-alcohol soluble extract of hardy kiwifruit**

Except using various ratio of water-alcohol solvent mixture such as 30 %, 50 %, and 70 % ethanol solvent with as an extraction solvent, all the procedure was identical to those of Example 1-1. As a result, 11 g ∼ 13 g of dried power of hardy kiwifruit were obtained at each ratio of solvent mixture and the dried powder was dissolved in distilled water (100 mg/ml).

**Example 2. Preparation of polar solvent and non-polar solvent soluble hardy kiwifruit extract**

The water extract prepared in Example 1-1 was subject to fractionation by following procedure.

**2-1. Preparation of chloroform soluble fraction (Comparitive example)**

50 ml of distilled water was added to 5 g of hardy kiwifruit extract obtained in Example 1-1. 50 ml of chloroform was added thereto in separatory funnel, shaken vigorously to divide into chloroform soluble layer and water soluble layer.

**2-2. Preparation of ethyl acetate soluble fraction**

Above water soluble layer obtained in Example 1-1 was mixed with 50 ml of ethyl acetate and then divided into ethyl acetate soluble layer and water soluble layer.

Above chloroform soluble layer, ethyl acetate soluble layer and water layer were concentrated by rotary evaporator, dried with freeze dryer to obtain 0.34 g of chloroform soluble fraction, 0.05 g of ethyl acetate soluble fraction and 4.61 g of water fraction powders respectively

**Example 3. Fractionation of hardy kiwifruit extract by silica gel column chromatography**

2,784 mg of ethyl acetate soluble fraction in Example 2-2 was further subjected to silica gel column chromatography (Daiso gel IR-60-W-40: 63 mm). The developing solvent was started with chloroform: methanol: water ([1] 90: 11: 1, [2] 60: 10: 1, [3] 60: 20: 2) solvent mixture and ended with methanol[4] with eluting speed of 300 ml/hr to obtain four sub-fractions([1] 2,381 mg, [2] 135 mg, [3] 148 mg, [4] 98 mg).

Above water extract, ethyl acetate soluble fraction and four sub-fractions were subjected to TLC (TLC plate: Merck Co. Ltd., Developing solvent; chloroform: methanol: water = 9: 5: 1) and the results were shown in Fig. 1. As shown in Fig. 1, lane 1 is water extracts, lane 2 is ethyl acetate soluble fractions, lane 3 is [4] sub-fraction, lane 4 is [3] sub-fraction, lane 5 is [2] sub-fraction and lane 6 is [1] sub-fractions.

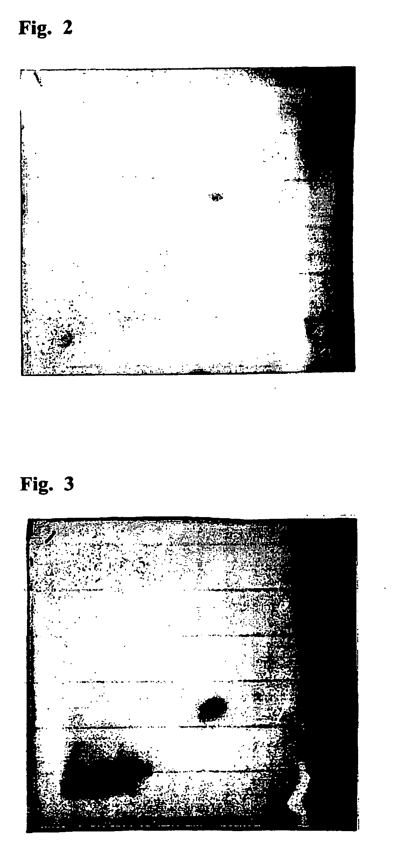

Above [1] and [2] sub-fractions were subjected to 2D-TLC using chloroform: methanol: water (9: 5: 1) solvent mixture as a 1^{st} developer and chloroform: acetone: water (3: 8: 0.5) solvent mixture as a 2^{rd} developer (*See* Fig. 1 and Fig. 2).

**Experimental Example 1. Inhibiting activity on the formation of blood DHT**

**1-1. Effect of polar soluble extract of hardy kiwi on the concentration of blood DHT**

To confirm the inhibition effect of hardy kiwifruit extract on the concentration of blood DHT, the water extract and ethanol extract of hardy kiwi were administrated into mice and the concentration of blood DHT were determined by following procedure;

M ale C57BU6 mice, aged 6 weeks (Seoul national university animal experimental center) were acclimated to the environment for 1 week. After 7 weeks, 16 mice were divided into 4 groups and each group was orally administered with 100ul of the hardy kiwifruit water extract (1500 µg /mouse/day), 100ul of the hardy kiwifruit ethanol extract (1500 µg /mouse/day), 100ul of finasteride (100 µg/mouse/day) and 100ul of drinking water (100µg/mouse/day) for 3 weeks, respectively After mice were sacrificed and the blood serum and spleen organ were colllected therefrom, the concentration of blood DHT was measured by ELISA(IBL-Hamburg GmbH) method.

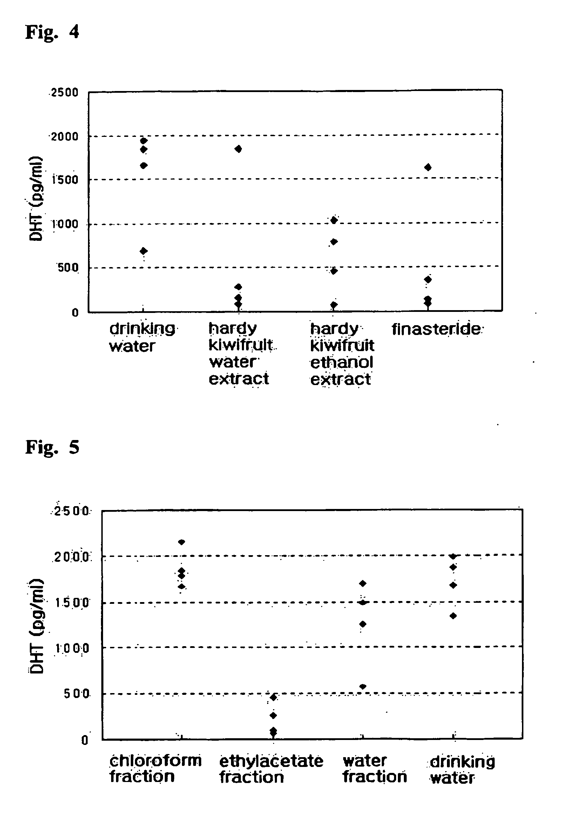

As shown in the Fig. 4, the concentration of blood DHT in groups administered with hardy kiwifruit extracts showed a decrease of about 90% with similar to groups administered with finasteride.

**1-2. Effect of non-polar soluble extract of hardy kiwi on the concentration of blood DHT**

To measure the concentration of blood DHT of C57BL/6 mice, chloroform soluble fraction, ethyl acetate soluble fraction and water soluble fraction prepared in above Example 2 were subjected to the identical experiment disclosed in above Experimental Example 1-1.

50µl of the chloroform soluble fraction, ethyl acetate soluble fraction and water soluble fraction were administrated to C57BL/6 mice, respectively. 100µl of drinking water was administrated as control.

As shown in the Fig. 5, the concentration of blood DHT showed a decrease of about 95% in the group administered with the ethylacetate doluble fraction of hardy kiwifruit and a slight decrease in the group administered with the water soluble fraction of hardy kiwifruit. However, it was not affected in the group administrated with the chloroform soluble fraction of hardy kiwifruit.

**1-3. Effect of silica gel column chromatography fraction on the concentration of blood DHT**

To measure the concentration of blood DHT of C57BL/6 mice, chloroform soluble fraction, silica gel column chromatography fraction [1], [2], [3] and [4] prepared in above Example 3 were subjected to the identical experiment disclosed in above Experimental Example 1-1.

30ul of s silica gel column chromatography fraction [1], [2], [3] and [4] were administrated to C57HL/6 mice, respectively 100ul of drinking water was administrated as control.

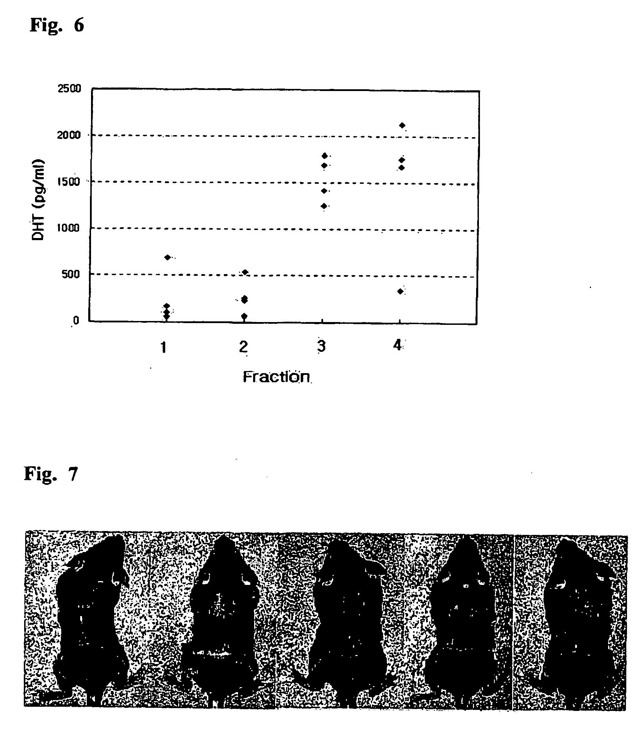

As shown in the Fig. 6, mouse administered with fraction [1] and [2] shows significantly inhibition of DHT.

**1-4. Effect of water soluble extract of hardy kiwifruit stem on the concentration of blood DHT**

To measure the concentration of blood DHT of C57BL/6 mice, water soluble fraction was subjected to the identical experiment disclosed in above Experimental Example 1-1.

After 7 weeks, 8 mice were divided into 2 groups and each group was orally administered with 100µl of the hardy kiwifruit stem water extract (1500µg /mouse/day) and 100ul of drinking water (100 µg mouse/day) for 3 weeks, respectively. After mice were sacrificed and the blood serum and spleen organ was collected therefrom, the concentration of blood DHT was measured. In the group administered with hardy kiwifruit stem water extract, the concentration of blood DHT was 142 pg/ml, 193 pg/ ml, 362 pg/ml and 1625 pg/ml, respectively Therefore, it confirmed that hardy kiwifruit stem extracts as well as hardy kiwifruit extracts showed a inhibition effect on DHT formation.

**Experimental Example 2. Stimulation effect on the formation of hair root**

**2-1. Effect of water soluble extract of hardy kiwifruit on the formation of hair root**

To confirm the effect of water soluble extract of hardy kiwifruit on the formation of hair root, mouse model was used as follows;

Fern ale C57BL/6 mice aged 6 weeks (Seoul national university animal experimental center) were acclimated to the environment for 7 days. After 7 weeks, the hair on the back of each mouse was carefully shaved using electrical shaver. Then 10 mice were divided into 2 groups and each group was orally administered with 100µl of the hardy kiwifruit water extract (1500 µg /mouse/day) and 100ul of drinking water (100 µg/mouse/day) for 4 weeks, respectively. Whether hair root was activated or not can be easily recognized by confirming the change of skin color where the hair was removed from bright red to darkened color by melanin pigment. Also, the growth of hair can be determined by observing directly the effect of water soluble extract of hardy kiwifruit on the formation of hair root.

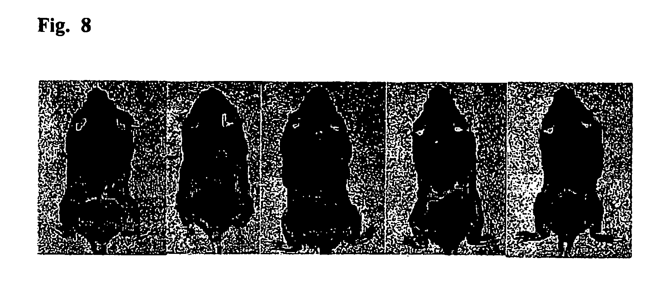

As shown in the Fig. 7 and 8, the skin color of hair removed region in the group administered with hardy kiwifruit water fraction, got darken within 2 weeks after the administration, which showed the growing of hair. However, the growth of hair as well as skin color did not showed in the group administrated hardy kiwifruit chloroform fraction. At the time of 4 weeks after adiministration, the back of each mouse in the former group was covered with new hair, while that in the latter group did not showed any new hair yet. Therefore, it confirmed that the the hardy kiwifruit extract can stimulate hair growth in the mechanism of promoting the formation of hair root of mouse.

**2-2. Effect of non-polar soluble extract of hardy kiwi on the formation of hair root**

To confirm the effect of chloroform soluble fraction, ethyl acetate soluble fraction and water soluble fraction which were prepared in above Example 2 on the formation of hair root, following experiments were subjected in similar procedure to that disclosed in above Experimental Example 2-1.

Then 20 mice were divided into 4 groups and each group was orally administered with 100µl of the hardy kiwifruit water fraction, 100µl of the hardy kiwifruit ethyl acetate fraction , 100µl of the hardy kiwifruit chloroform fraction and 100µl of drinking water for 4 weeks, respectively.

At the result, hair growth was highly stimulated significantly in the group administered with the ethyl acetate soluble fraction of hardy kiwifruit compared with those of the other groups.

**Experimental Example 3. Effect of hardy kiwifruit extract on the improvement of seborrheic skin disorders**

To test the effect of hardy kiwifruit extract on the improvement of seborrheic skindisorders, each 1g of hardy kiwifruit water extract were administered orally once a day for 4 weeks to three volunteers suffered from seborrheic dermatitis to confirm whether the seborrheic skin disorder were improved or not as follows:

3-1. Patient K, a 29 years old Korean female who had been suffered from severe skin seborrheication and lots of dandruffs to progress to depilation. After she had taken tablets containing 1g of hardy kiwifruit water extract once a day for 2 weeks, the seborrheic and keratogenous skin disorders was significantly reduced and the depilation syndrome were reduced to more than about 50%.

3-2. Patient P, a 27 years old Korean male who had been suffered from oily face and saclp and lots of dandruffs. After he had taken tablets containing 1g of hardy kiwifruit water extract once a day for 2 weeks, the oily face was significantly alleviated and the alopecia was reduced to more than about 70%.

3-2. Patient U, a 29 years old Korean male who had been suffered from seborrheic skin, especially, face and purutitic and spotty scalp from several years ago. After he had taken tablets containing 1g of hardy kiwifruit water extract once a day for 2 weeks, the purutitic syndrome of scalp was disappeared and the trouble caused by oily face, for example, pimple, was also disappeared significantly.

**Experimental Example 4. Toxicity Test**

To examine the toxicity of the hardy kiwifruit extract, repetitive toxicity tests were performed on mouse.

The 10 female of Balb/c mice were divided with 2 groups and inventive hardy kiwifruit extract (150 mg/kg) was administered to the mice at 150 mg/kg for 4 weeks and water was treated to the control group. The symptom of toxicity was observed for 4 weeks such as the change of weight, the hematological analysis and histological test.

As a result of experiment, there was no death example of the mice administered with 150 mg/kg inventive hardy kiwifruit and there was no significant abnormality in the gain of weight, the caloric intake of feed, the hematological analysis and the histological test etc. In accordance with above results, it was confirmed that the hardy kiwifruit was safe medicine.

(1) Weight and observation: the unusual change of weight was not observed.

(2) Hematological analysis: No abnormal symptom was observed in the number of WBC, lymphocyte, monocyte, neutrophil, eosinophil, basophil, RBC, hemoglobin and platelet.

(3) Serum biochemical test: No abnormal symptom was observed in the level of AST, ALT, LDH, bilirubin, creatinine, glucose, cholesterol, minerals, albumin, BUN, lipase and amylase of serum.

(4) Histological test: No abnormal symptom was observed in the tissue of kidneys, the spleen, the liver and the thymus.

Hereinafter, the formulating methods and kinds of exipients will be described, but the present invention is not limited to them. The representative preparation examples were described as follows.

Preparation of injection

| | |
|---|---|
| Hardy kiwifruit water extract of Example 1 | 50 mg |
| Sodium metadisulfite | 3.0 mg |
| Methylparaben | 0.8 mg |
| Propylparaben | 0.1 mg |
| Distilled water for injection | optimum amount |

Injection preparation was prepared by mixing above components and making 2 ml by the conventional method and then filing filling all the components 2 ml ample and sterilizing by conventional injection preparation method.

Preparation of tablet

| | |
|---|---|
| Hardy kiwifruit water extract of Example 1 | 50 mg |
| Corn Starch | 100 mg |
| Lactose | 100 mg |
| Magnesium Stearate | 2 mg |

Tablet preparation was prepared by mixing above components and entabletting.

Preparation of capsule

| | |
|---|---|
| Hardy kiwifruit water extract of Example 1 | 100 mg |
| Corn starch | 100 mg |
| Lactose | 100 mg |
| Talc | 2 mg |
| Magnesium Stearate | optimum amount |

Tablet preparation was prepared by mixing above components and filling gelatin capsule by conventional gelatin preparation method.

Preparation of liquid

| | |
|---|---|
| Hardy kiwifruit water extract of Example 1 | 100 mg |
| Sugar | 20 g |
| Fructose | 20 g |
| Lemon flavour | optimum amount |
| Distilled water | 100 ml |

Liquid preparation was prepared by mixing above components and then filling 100 me brown bottle sterilizing by conventional liquid preparation method.

Preparation of health care food

| | |
|---|---|
| Hardy kiwifruit water extract of Example 1 | 1000 mg |
| Vitamin mixture | 20 g |
| vitamin A acetate | 70µg |
| Vitamin E | 1.0 mg |
| Vitamin B ₁ | 0.13 mg |
| Vitamin B ₂ | 0.15 mg |
| Vitamin B ₆ | 0.5 mg |
| Vitamin B ₁₂ | 0.2 µg |
| Vitamin C | 10 mg |
| Biotin | 1Cµg |
| Amide nicotinic acid | 1.7 mg |
| Folic acid | 50 µg |
| Calcium pantothenic acid | 0.5 mg |
| Mineral mixture | optimum amount |
| Ferrous sulfate | 1.75 mg |
| Zinc oxide | 0.82 mg |
| Magnesium carbonate | 25.3 mg |
| Monopotassium phosphate | 15 mg |
| Dicalcium phosphate | 55 mg |
| Potassium citrate | 90 mg |
| Calcium carbonates | 100 mg |
| Magnesium chloride | 24.8 mg |

The above-mentioned vitamin and mineral mixture may be varied in many ways. Such variations are not to be regarded as a departure from the spirit and scope of the present invention.

Preparation of health beverage

| | |
|---|---|
| Hardy kiwifruit water extract of Example 1 | 1000 mg |
| Citric acid | 100 mg |
| Oligosaccharide | 100 g |
| Apricot concentration | 2 g |
| Taurine | 1 g |
| Distilled water | 900 ml |

Health beverage preparation was prepared by dissolving active component, mixing, stirred at 85 °C for 1 hour, filtered and then filling all the components in 2000 ml ample and sterilizing by conventional health beverage preparation method.

Preparation of skin lotion

| | |
|---|---|
| Hardy kiwifruit water extract of Example 1 | 1.00(%) |
| Glycerol | 3.00 |
| Ethanol | 1.00 |
| Propylene glycol | 0.10 |
| Flavour | trace amount |
| Distilled water | made to 100% |

Skin preparation was prepared by dissolving active component according to conventional lotion preparation method.

Preparation of lotion

| | |
|---|---|
| Hardy kiwifruit water extract of Example 1 | 3.00(%) |
| L-ascorbic acid-2-magnesium phosphate | 1.00 |
| Soluble collagen (1% solution) | 1.00 |
| Sodium citric acid | 0.10 |
| Citric acid | 0.05 |
| 1,3-butylene glycol | 3.00 |
| Distilled water | made to 100% |

Lotion preparation was prepared by dissolving active component according to conventional lotion preparation method.

Preparation of cream

| | |
|---|---|
| Hardy kiwifruit water extract of Example 1 | 3.00(%) |
| Polyethyleneglycomonosterate | 2.00 |
| Monostearate glycerin | 1.00 |
| Cetyl alcohol | 4.00 |
| Squalene | 6.00 |
| Tri 2- glyceryl ethylhexanoate | 6.00 |
| Sphingo-glycolipid | 1.00 |
| 1,3-butylene glycol | 7.00 |
| Distilled water | made to 100% |

Preparation of pack

| | |
|---|---|
| Hardy kiwifruit water extract of Example 1 | 5.00(%) |
| Polyvinyl alcohol | 13.00 |
| L-ascorbic acid-2-magnesium phosphate | 1.00 |
| Lauroylhydroxyproline | 1.00 |
| Soluble collagen (1% solution) | 2.00 |
| 1,3-butylene glycol | 3.00 |
| Ethanol | 5.00 |
| Distilled water | made to 100% |

Pack preparation was prepared by dissolving active component according to conventional pack preparation method.

Preparation of beauty solution

| | |
|---|---|
| Hardy kiwifruit water extract of Example 1 | 2.00(%) |
| Hydroxyethylenecellulose (2% solution) | 12.00 |
| Xanthin gum (2% solution) | 2.00 |
| 1,3-butylene glycol | 3.00 |
| Conc. Glycerin | 4.00 |
| Sodium hyaluronate | 5.00 |
| Distilled water | made to 100% |

Beauty solution preparation was prepared by dissolving active component according to conventional beauty solution preparation method.

## Claims

1. A use of a crude or purified extract of the hardy kiwifruit for the preparation of a therapeutic agent for treating and preventing a disease selected from alopecia, seborrheic dermatitis, keratogenous skin disorder and pruritic scalp in a human or a mammal, wherein the crude or purified extract is soluble in water, lower alcohol such as methanol, ethanol, and butanol or mixtures thereof or in ethyl acetate.

2. The use according to claim 1, wherein said hardy kiwifruit is selected from *Actinidia arguta*, *Actinidia kolomikta* and *Actinidia polygama.*

3. The use according to claim 1, wherein said extract of hardy kiwifruit is from any of fruit, stem or root thereof.

4. The use according to claim 1, wherein said purified extract is any of the fractions obtained by column chromatographic purification.

5. The use according to claim 1, wherein the amount of said extract ranges from 0.01 to 50 % by weight based on the total weight of the agent.

6. A pharmaceutical composition comprising a crude or purified extract of the hardy kiwifruit as an active ingredient for use in the treatment and prevention of a disease selected from alopecia, seborrheic dermatitis, keratogenous skin disorder and pruritic scalp, wherein the crude or purified extract is soluble in water, lower alcohol such as methanol, ethanol, and butanol or mixtures thereof or in ethyl acetate.

7. The pharmaceutical composition for use according to claim 6, wherein said composition is skin lotion, cream, essence, toner, emulsion, pack, soap, shampoo, rinse, cleanser, body washing solution, washing solution or treatment.

8. A health care food comprising a crude or purified extract of the hardy kiwifruit as an active ingredient together with a sitologically acceptable additive for use in the prevention and improvement of a disease selected from alopecia, seborrheic dermatitis, keratogenous skin disorder and pruritic scalp, wherein the crude or purified extract is soluble in water, lower alcohol such as methanol, ethanol, and butanol or mixtures thereof or in ethyl acetate.

9. The health care food for use according to claim 8 wherein said hardy kiwifruit is selected from *Actinidia arguta, Actinidia kolomikta* and *Actinidia polygama.*

10. The health food for use according to claim 8 wherein said extract of hardy kiwifruit is produced from any of fruit, stem or root thereof.

11. The health care food for use according to claim 8 wherein the amount of said extract comprises 0.01 to 80 % by weight based on the total weight of the composition.

12. The health care food for use according to claim 8 wherein said health care food is provided as powder, granule, tablet, capsule or beverage.

13. A food additive comprising a crude extract of hardy kiwifruit as an active ingredient for use in the prevention and improvement of a disease selected from alopecia, seborrheic dermatitis, keratogenous skin disorder and pruritic scalp, wherein the crude extract is soluble in water, lower alcohol such as methanol, ethanol, and butanol or mixtures thereof or in ethyl acetate.

14. The food additive for use according to claim 13 wherein said crude extract of hardy kiwifruit further comprises at least one compound selected from lactose, casein, dextrin, glucose, sucrose and sorbitol.

15. The food additive for use according to claim 13 wherein the ratio of said additives is ranging from about 0.01 to 20 w/w % per 100 w/w % of the present composition.

16. The food additive for use according any one of claims 13 to 15 wherein said food additive is used as a spice, seasoning or food material.

17. The food additive for use according to claim 13 wherein said food additive can be added to food by deposition, spraying or mixing.

18. The food additive for use according to claim 13 wherein said food is at least one selected from fruits, vegetables, dehydrated foods or cutting products such as fruits, vegetables; fruit juice, vegetable juices or a mixture thereof; drinks containing acid-beverage; confectionaries such as cookie, candy, caramel, gum; breads; ice creams, teas, fermented milk such as yogurt; dairy product, spices, alcoholic beverages, cans, in-bottles, noodles, processed livestock products, processed marine products, fermented food, beans food, cereals food, processed meats, licorices and herbs.

19. A feed additive comprising a crude extract of hardy kiwifruit as an active ingredient for use in the prevention and treatment of alopecia and seborrheic dermatitis, keratogenous skin disorder and pruritic scalp, wherein said crude extract is soluble in polar solvent selected from distilled water, lower alcohols such as ethanol, methanol, butanol, or the mixtures thereof.

20. The feed additive for use according to claim 19 wherein said hardy kiwifruit is selected from *Actinidia arguta, Actinidia kolomikta* and *Actinidia polygama.*

21. The feed additive for use according to claim 19 wherein said feed additive is provided as liquid, powder, or granule.

22. The feed additive for use according to claim 19 wherein said feed additive can be added to feed by deposition, spray, or mixing.

23. A feed composition comprising any one of feed additives for use according to claims 19 to 22.

## Patentansprüche

1. Verwendung eines rohen oder gereinigten Extrakts der winterharten Kiwi zur Herstellung eines therapeutischen Mittels zur Behandlung oder Vorbeugung einer Krankheit ausgewählt aus Alopecia, seborrhoischer Dermatitis, keratogener Hauterkrankung und juckender Kopfhaut bei einem Menschen oder einem Säuger, wobei der rohe oder gereinigte Extrakt löslich ist in Wasser, niedrigen Alkoholen wie Methanol, Ethanol und Butanol oder Mischungen davon, oder in Ethylacetat.

2. Verwendung nach 1, wobei die winterharte Kiwi ausgewählt ist aus *Actinidia arguta, Actinidia kolomikta* und *Actinidia polygama.*

3. Verwendung nach Anspruch 1, wobei der Extrakt der winterharten Kiwi von der Frucht, dem Stamm oder den Wurzeln stammt.

4. Verwendung nach Anspruch 1, wobei der Extrakt aus einer der Fraktionen der Säulenchromatographiereinigung erhalten wird.

5. Verwendung nach Anspruch 1, wobei die Menge des Extraktes im Bereich von 0,01 bis 50 Gewichtsprozent basierend auf dem Gesamtgewicht des Mittels liegt.

6. Pharmazeutische Zusammensetzung umfassend einen rohen oder gereinigten Extrakt der winterharten Kiwi als Wirkstoff zur Verwendung bei der Behandlung und Vorbeugung einer Krankheit ausgewählt aus Alopecia, seborrhoische Dermatitis, keratogener Hauterkrankung und juckender Kopfhaut, wobei der rohe oder gereinigte Extrakt löslich ist in Wasser, niedrigen Alkoholen wie Methanol, Ethanol und Butanol oder Mischungen davon, oder in Ethylacetat.

7. Pharmazeutische Zusammensetzung zur Verwendung nach Anspruch 6, wobei die Zusammensetzung eine Hautlotion, Krem, Essenz, Toner, Emulsion, Packung, Seife, Shampoo, Spülung, Reiniger, Körperwaschlösung, Reinigungslösung oder Reinigungsbehandlung ist.

8. Gesundheitsnahrung umfassend einen rohen oder gereinigten Extrakt der winterharter Kiwi als Wirkstoff zusammen mit einem sitologisch verträglichen Zusatzstoff zur Verwendung bei der Vorbeugung oder Verbesserung einer Krankheit ausgewählt aus Alopecia, seborrhoische Dermatitis, keratogener Hauterkrankung und juckender Kopfhaut, wobei der rohe oder gereinigte Extrakt löslich ist in Wasser, niedrigen Alkoholen wie Methanol, Ethanol und Butanol oder Mischungen davon, oder in Ethylacetat.

9. Gesundheitsnahrung zur Verwendung nach Anspruch 8, wobei die winterharte Kiwi ausgewählt ist aus *Actinidia arguta, Actinidia kolomikta* und *Actinidia polygama.*

10. Gesundheitsnahrung zur Verwendung nach Anspruch 8, wobei der Extrakt der winterharten Kiwi aus der Frucht, dem Stamm oder den Wurzeln hergestellt wird.

11. Gesundheitsnahrung zur Verwendung nach Anspruch 8, wobei die Menge des Extrakts im Bereich von 0,01 bis 80 Gewichtsprozent basierend auf dem Gesamtgewicht der Zusammensetzung liegt.

12. Gesundheitsnahrung zur Verwendung nach Anspruch 8, wobei die Gesundheitsnahrung als Pulver, Granulat, Tablette, Kapsel oder Getränk bereitgestellt wird.

13. Nahrungsmittelzusatz, umfassend einen rohen oder gereinigten Extrakt der winterharter Kiwi als Wirkstoff zur Verwendung bei der Vorbeugung und der Verbesserung einer Krankheit ausgewählt aus Alopecia, seborrhoischer Dermatitis, keratogener Hauterkrankung und juckender Kopfhaut, wobei der rohe oder gereinigte Extrakt löslich ist in Wasser, niedrigen Alkoholen wie Methanol, Ethanol und Butanol oder Mischungen davon, oder in Ethylacetat.

14. Nahrungsmittelzusatz zur Verwendung nach Anspruch 13, wobei der rohe Extrakt der winterharten Kiwi weiterhin mindestens eine Verbindung ausgewählt aus Laktose, Casein, Dextrin, Glucose, Saccharose und Sorbitol umfasst.

15. Nahrungsmittelzusatz zur Verwendung nach Anspruch 13, wobei das Verhältnis des Zusatzes im Bereich von etwa 0,01 bis 20 Gew. % pro 100 Gew. % der vorliegenden Zusammensetzung liegt.

16. Nahrungsmittelzusatz zur Verwendung nach einem der Ansprüche 13 bis 15 wobei der Nahrungsmittelzusatz als Gewürz, Würzmittel oder Nahrungsmittel verwendet wird.

17. Nahrungsmittelzusatz zur Verwendung nach Anspruch 13, wobei der Nahrungsmittelzusatz dem Nahrungsmittel durch Ablagern, Zerstäuben oder Mischen zugesetzt werden kann.

18. Nahrungsmittelzusatz zur Verwendung nach Anspruch 13, wobei das Nahrungsmittel mindestens eines ist, ausgewählt aus: Früchten, Gemüse, Trockenfrüchten oder Schnittprodukten wie Früchte, Gemüse; Fruchtsaft, Gemüsesäfte oder eine Mischung davon; Getränke, die Säure enthalten; Süßspeisen wie Kekse, Süßigkeiten, Karamell, Gummi; Brote; Eiskrems, Tees, fermentierte Milch wie Yoghurt; Diätprodukte, Gewürze, alkoholische Getränke, Konserven, Produkte in Flaschen, Nudeln, verarbeitete tierische Erzeugnisse, verarbeitete Meereserzeugnisse, fermentierte Lebensmittel, Lebensmittel aus Bohnen, Lebensmittel aus Getreide, verarbeitete Fleischwaren, Lakritze und Kräuter.

19. Futtermittelzusatz umfassend einen rohen oder gereinigten Extrakt der winterharter Kiwi als Wirkstoff zur Verwendung bei der Vorbeugung und der Behandlung von Alopecia, seborrhoischer Dermatitis, keratogener Hauterkrankung und juckender Kopfhaut, wobei der rohe oder gereinigte Extrakt löslich ist in polaren Lösungsmitteln ausgewählt aus destilliertem Wasser, niedrigen Alkoholen wie Ethanol, Methanol und Butanol oder Mischungen davon.

20. Futtermittelzusatz zur Verwendung nach Anspruch 19, wobei die winterharte Kiwi ausgewählt ist aus *Actinidia arguta, Actinidia kolomikta* und *Actinidia polygama.*

21. Futtermittelzusatz zur Verwendung nach Anspruch 19, wobei der Futtermittelzusatz als Flüssigkeit, Pulver oder Granulat bereitgestellt wird.

22. Futtermittelzusatz zur Verwendung nach Anspruch 19, wobei der Futtermittelzusatz dem Futtermittel durch Ablagern, Zerstäuben oder Mischen zugesetzt werden kann.

23. Futtermittelzusammensetzung umfassend einen der Futtermittelzusätze zur Verwendung nach einem der Ansprüche 19 bis 22.

## Revendications

1. Utilisation d'un extrait brut ou purifié de kiwi rustique pour la préparation d'un agent thérapeutique destiné à traiter et à prévenir une maladie choisie parmi l'alopécie, la dermatite séborrhéique, les troubles cutanés kératogènes et le cuir chevelu prurigineux chez un humain ou un mammifère, dans laquelle l'extrait brut ou purifié est soluble dans l'eau, les alcools inférieurs tels que le méthanol, l'éthanol et le butanol ou leurs mélanges, ou dans l'acétate d'éthyle.

2. Utilisation selon la revendication 1, dans laquelle ledit kiwi rustique est choisi parmi *Actinidia arguta, Actinidia kolomikta* et *Actinidia polygama.*

3. Utilisation selon la revendication 1, dans laquelle ledit extrait de kiwi rustique est un extrait de l'un quelconque parmi son fruit, sa tige et sa racine.

4. Utilisation selon la revendication 1, dans laquelle ledit extrait purifié est l'une quelconque des fractions obtenues par purification chromatographique sur colonne.

5. Utilisation selon la revendication 1, dans laquelle la quantité dudit extrait est située dans la plage allant de 0,01 à 50 % en poids par rapport au poids total de l'agent.

6. Composition pharmaceutique comprenant un extrait brut ou purifié de kiwi rustique à titre d'ingrédient actif, pour utilisation dans le traitement et la prévention d'une maladie choisie parmi l'alopécie, la dermatite séborrhéique, les troubles cutanés kératogènes et le cuir chevelu prurigineux, dans laquelle l'extrait brut ou purifié est soluble dans l'eau, les alcools inférieurs tels que le méthanol, l'éthanol et le butanol ou leurs mélanges, ou dans l'acétate d'éthyle.

7. Composition pharmaceutique pour utilisation selon la revendication 6, laquelle composition est une lotion pour la peau, une crème, une essence, un tonique, une émulsion, un masque, un savon, un shampooing, un après-shampooing à rincer, un nettoyant, une solution lavante pour le corps, une solution lavante ou un traitement.

8. Aliment de santé comprenant un extrait brut ou purifié du kiwi rustique à titre d'ingrédient actif, conjointement avec un additif acceptable du point de vue sitologique, pour utilisation dans la prévention et l'amélioration d'une maladie choisie parmi l'alopécie, la dermatite séborrhéique, les troubles cutanés kératogènes et le cuir chevelu prurigineux, dans lequel l'extrait brut ou purifié est soluble dans l'eau, les alcools inférieurs tels que le méthanol, l'éthanol et le butanol ou leurs mélanges, ou dans l'acétate d'éthyle.

9. Aliment de santé pour utilisation selon la revendication 8, dans lequel ledit kiwi rustique est choisi parmi *Actinidia arguta, Actinidia kolomikta* et *Actinidia polygama.*

10. Aliment de santé pour utilisation selon la revendication 8, dans lequel ledit extrait de kiwi rustique est un extrait de l'un quelconque parmi son fruit, sa tige et sa racine.

11. Aliment de santé pour utilisation selon la revendication 8, dans lequel la quantité dudit extrait représente de 0,01 à 80 % en poids par rapport au poids total de la composition.

12. Aliment de santé pour utilisation selon la revendication 8, lequel aliment de santé se présente sous la forme d'une poudre, de granules, d'un comprimé, d'une capsule ou d'une boisson.

13. Additif alimentaire comprenant un extrait brut de kiwi rustique à titre d'ingrédient actif, pour utilisation dans la prévention et l'amélioration d'une maladie choisie parmi l'alopécie, la dermatite séborrhéique, les troubles cutanés kératogènes et le cuir chevelu prurigineux, dans lequel l'extrait brut est soluble dans l'eau, les alcools inférieurs tels que le méthanol, l'éthanol et le butanol ou leurs mélanges, ou dans l'acétate d'éthyle.

14. Additif alimentaire pour utilisation selon la revendication 13, dans lequel ledit extrait brut de kiwi rustique comprend en outre au moins un composé choisi parmi le lactose, la caséine, la dextrine, le glucose, le saccharose et le sorbitol.

15. Additif alimentaire pour utilisation selon la revendication 13, dans lequel le rapport desdits additifs est situé dans la plage allant d'environ 0,01 à 20 % en poids pour 100 % en poids de la présente composition.

16. Additif alimentaire pour utilisation selon l'une quelconque des revendications 13 à 15, lequel additif alimentaire est utilisé en tant qu'épice, assaisonnement ou matière alimentaire.

17. Additif alimentaire pour utilisation selon la revendication 13, lequel additif alimentaire peut être ajouté à un aliment par dépôt, pulvérisation ou mélange.

18. Additif alimentaire pour utilisation selon la revendication 13, dans lequel ledit aliment est au moins un aliment choisi parmi les fruits, les légumes, les aliments déshydratés ou les produits en morceaux tels que les fruits et les légumes ; le jus de fruit, les jus de légumes ou leurs mélanges ; les boissons contenant un acide à boire ; les confiseries telles que les cookies, les bonbons, le caramel, la gomme ; les pains ; les crèmes glacées, les thés, le lait fermenté tel que le yaourt ; les produits laitiers, les épices, les boissons alcoolisées, les conserves, les produits en bouteille, les nouilles, les produits carnés transformés, les produits de la mer transformés, les aliments fermentés, les aliments à base de haricot, les aliments céréaliers, les viandes transformées, la réglisse et les herbes.

19. Additif alimentaire pour animaux comprenant un extrait brut de kiwi rustique à titre d'ingrédient actif, pour utilisation dans la prévention et l'amélioration d'une maladie choisie parmi l'alopécie, la dermatite séborrhéique, les troubles cutanés kératogènes et le cuir chevelu prurigineux, dans lequel l'extrait brut est soluble dans un solvant polaire choisi parmi l'eau distillée, les alcools inférieurs tels que le méthanol, l'éthanol et le butanol, ou leurs mélanges.

20. Additif alimentaire pour animaux pour utilisation selon la revendication 19, dans lequel ledit kiwi rustique est choisi parmi *Actinidia arguta, Actinidia kolomikta* et *Actinidia polygama.*

21. Additif alimentaire pour animaux pour utilisation selon la revendication 19, lequel additif alimentaire pour animaux se présente sous la forme d'un liquide, d'une poudre, ou de granules.

22. Additif alimentaire pour animaux pour utilisation selon la revendication 19, lequel additif alimentaire pour animaux peut être ajouté à l'aliment pour animaux par dépôt, pulvérisation, ou mélange.

23. Composition alimentaire pour animaux comprenant l'un quelconque des additifs alimentaires pour animaux pour utilisation selon les revendications 19 à 22.
